# EUROPÄISCHE PATENTSCHRIFT

(11) **EP 3 615 091 B1**
(45) Veröffentlichungstag und Bekanntmachung des Hinweises auf die Patenterteilung: **28.10.2026**
(21) Anmeldenummer: 18724146.8
(22) Anmeldetag: 24.04.2018
(51) Int. Cl.: A61L 2/07

(54) **ENTNAHMEVORRICHTUNG ZUR ENTNAHME VON FLÜSSIGKEITEN ZUR HERSTELLUNG PARENTERALER ARZNEIMITTEL AUS EINEM LEITUNGSSYSTEM**
RETRIEVAL DEVICE FOR RETRIEVING LIQUIDS FOR THE PRODUCTION OF PARENTERAL MEDICAMENTS FROM DUCTWORK
DISPOSITIF DE PRÉLÈVEMENT À PARTIR D'UN SYSTÈME DE CONDUITE DE LIQUIDES DESTINÉS À FABRIQUER UN MÉDICAMENT À ADMINISTRER PAR VOIE PARENTÉRALE

(30) Priorität: 24.04.2017 EP 17167704
(43) Veröffentlichungstag der Anmeldung: 04.03.2020
(73) Patentinhaber: F. Hoffmann-La Roche AG, 4070 Basel (CH)
(72) Erfinder: BENDEL, Volker, 68305 Mannheim (DE); LAIKA, Matthias, 68305 Mannheim (DE); RIEGER, Wolfgang, 68305 Mannheim (DE)
(74) Vertreter: Altmann Stößel Dick Patentanwälte PartG mbB
(86) Internationale Anmeldenummer: PCT/EP2018/060403
(87) Internationale Veröffentlichungsnummer: WO 2018/197446

(56) Entgegenhaltungen:
- EP-A1- 0 487 214
- EP-A1- 1 852 131
- WO-A1-03/029721
- WO-A1-2005/016814
- DE-A1- 102005 023 391
- DE-A1- 2 755 155
- US-A- 3 870 033
- US-A- 4 547 339
- US-A- 5 290 442
- U. BOBE ET AL: "Anforderungen an Werkstoffe und Werkstoffoberflächen bezüglich Reinigbarkeit und Beständigkeit", CHEMIE INGENIEUR TECHNIK., vol. 78, no. 11, 1 November 2006 (2006-11-01), WEINHEIM; DE, pages 1615 - 1622, XP055423970, ISSN: 0009-286X, DOI: 10.1002/cite.200600095

## Beschreibung

### Gebiet der Erfindung

Die Erfindung betrifft ein Leitungssystem zur Bereitstellung von keimarmer Flüssigkeit, insbesondere pharmazeutischem Wasser sowie ein Verfahren zur Entnahme von keimarmer Flüssigkeit, insbesondere pharmazeutischem Wasser, aus einem Leitungssystem. Die Vorrichtung und das Verfahren gemäß der vorliegenden Erfindung lassen sich insbesondere in der pharmazeutischen Industrie einsetzen, beispielsweise zur Herstellung von Infusion- oder Injektionslösungen. Die Vorrichtung und das Verfahren gemäß der vorliegenden Erfindung lassen sich insbesondere für "Water for Injection" (Wasser für Injektionszwecke, WFI), "Highly Purified Water" (Hochreines Wasser, HPW) oder auch allgemein für Reinwasser und Reinstwasser einsetzen. Insbesondere kann Wasser der Qualität "WFI", "HPW" oder "AP" (Aqua Purificata) nach dem Europäischen Arzneibuch eingesetzt werden, beispielsweise nach der PH. Eur. 8. Ausgabe Band 3, Seiten 5248- 5257. Auch andere Einsatzgebiete sind möglich.

### Technischer Hintergrund

In vielen Bereichen der Technik und Naturwissenschaften ist die Bereitstellung keimarmer, insbesondere steriler, Flüssigkeiten essenziell. Ohne Beschränkung weiterer möglicher Einsatzgebiete wird die Erfindung im Folgenden beschrieben unter Bezugnahme auf die Bereitstellung von sterilem Wasser für Injektionszwecke (WFI). Daneben existieren jedoch zahlreiche weitere Einsatzzwecke, beispielsweise in der Labordiagnostik oder in der biochemischen Forschung, in welchen sterile Flüssigkeiten verwendet werden.

Bei der Herstellung von so genannten Parenteralia, also Infusion- oder Injektionslösungen, wird in der Regel Wasser, auch als "Wasser für Injektionszwecke", "Water for Injection" oder WFI bezeichnet, verwendet. Zur Herstellung dieses Wassers werden verschiedene Aufbereitungstechniken, häufig in Kombination, eingesetzt, insbesondere Destillationsverfahren, Umkehrosmose oder Ultrafiltration. Das Wasser wird in der Regel in Tanks aufbewahrt, regelmäßig umgepumpt und über entsprechende Leitungssysteme am Einsatzort bereitgestellt.

In der DE 27 55 155 A1 wird eine Vorrichtung zum Reinigen von Zapfanlagen mit mindestens einem Kompensator-Zapfhahn offenbart. Die Vorrichtung ist gekennzeichnet durch einen Vorratsbehälter für eine Reinigungsflüssigkeit mit mindestens einem Auslaufstutzen und einem Verbindungsschlauch zum Auslauf des Kompensator-Zapfhahnes sowie durch ein Drosselorgan, welches an das dem Kompensator-Zapfhahn abgewandte Ende der Zapfleitung anschließbar ist.

Weiterhin beschreibt die WO 2005/016814 A1 eine Zapfanlage für Getränke, insbesondere Bier, sowie ein Verfahren zu deren Reinigung. Die Zapfanlage enthält einen Getränkebehälter, einen oberhalb des Getränkebehälters befindlichen und von diesem beabstandeten Zapfhahn, sowie eine Getränkeleitung, deren erstes, oberes Ende an den Zapfhahn und deren zweites, unteres Ende an den Getränkebehälter angeschlossen ist. Ferner ist eine Reinigungsanlage zum Einleiten eines Reinigungsmittels in die Getränkeleitung vorgesehen. Die Reinigungsanlage weist am ersten Ende der Getränkeleitung einen Zufluss und am zweiten Ende der Getränkeleitung einen Abfluss für das Reinigungsmittel auf.

Ferner offenbart die WO 2012/066626 A1 einen Spender umfassend ein Kaltwasser-Installationssystem zum Bereitstellen von gekühltem Wasser und ein Heißwasser-Installationssystem zum Bereitstellen von erwärmtem Wasser, wobei beide Systeme mittels eines Dreiwegeventils mit einer Einfüllöffnung verbunden sind, wobei ein Dreiwegeventil ein Ventil mit drei Anschlüssen ist. Der Spender ist charakterisiert durch einen Sterilisationsprozess, der durchgeführt wird, durch Beaufschlagen des Kaltwasser-Installationssystem und des Heißwasser-Installationssystem mit Wasser mittels des Dreiwegeventils und durch Zirkulation von heißem Wasser durch beide Installationssysteme unter Ausnutzung eines Konvektionsphänomens, welches auf dem Unterschied der Wassertemperaturen beruht. Der Spender ist in der Lage mithilfe einer sehr einfachen Struktur, in der lediglich ein Dreiwegeventil zusätzlich benutzt wird, thermische Sterilisation durchzuführen. Eine Kaltwassereinfüllöffnung, in welcher die Verbreitung von Bakterien am wahrscheinlichsten geschieht, kann mithilfe von Hitze sterilisiert werden unter Benutzung der Einfüllöffnung sowohl für kaltes als auch für heißes Wasser.

Weiter beschreibt die DE 10 2005 023391 A1 eine Vorrichtung zur thermischen Desinfektion von Atemgas führenden Baueinheiten eines Atemsystems mit einer an die Baueinheit koppelbaren Trageinheit, enthaltend mindestens eine Luftaufbereitungskomponente für die Desinfektion der Baueinheit, enthaltend eine Lufteintrittsöffnung und eine Luftaustrittsöffnung, so dass die Baueinheit im Desinfektionsbetrieb mit einem auf eine vorgegebene Mindesttemperatur erwärmten Luftstrom beaufschlagt ist, wobei die Lufteintrittsöffnung der Trageinheit an eine vorhandene Luftaustrittsöffnung der Baueinheit und die Luftaustrittsöffnung der Trageinheit an eine vorhandene Lufteintrittsöffnung der Baueinheit anschließbar ist, und dass die Baueinheit über mindestens eine Luftaufbereitungskomponente verfügt, so dass im Desinfektionsbetrieb die erwärmte Luft in einem durch die Trageinheit und die Baueinheit gebildeten Kreislauf zirkuliert.

Ferner offenbart die DE 32 38 434 A1 ein Verfahren und eine Vorrichtung zur Wärmebehandlung von Gütern umfassend die axiale Durchführung des zu behandelnden Gutes durch einen Ringraum, dessen äußere und innere Begrenzungsfläche thermostatisch gesteuert beheizt sind. Die innere Begrenzungsfläche wird durch einen als Schnecke ausgebildeten, motorisch antreibbaren Innenkörper gebildet, während die äußere Begrenzungsfläche durch ein Doppelmantelrohr dargestellt wird, das auf der Innenseite mit Zügen versehen ist. Das auf seiner Außenfläche isolierte äußere Rohr ist mit an dem Zwischenraum zwischen den Mantelflächen angeschlossenen Dampfzuführungs- und Kondensatableitungsöffnungen versehen. Der sich drehende Innenkörper ist mit einer Kondensathebevorrichtung versehen.

Weiterhin beschreiben Bobe et al. ("Anforderungen an Werkstoffe und Werkstoffoberflächen bezüglich Reinigbarkeit und Beständigkeit", CHEMIE INGENIEUR TECHNIK., BD. 78, NR. 11, 1. November 2006 (2006-11-01), Seiten 1615-1622, XP055423970, Weinheim; DE, ISSN: 0009-286X, DOI: 10.1002/C.200600095) den aktuellen Stand der Forschung hinsichtlich der Auswirkungen der Werkstoffoberflächen auf die Reinigbarkeit. Des Weiteren werden Ursachen für mögliche Korrosionsschäden an nichtrostenden Edelstählen einschließlich der Möglichkeiten zur Schadensverhütung sowie der Einsatzbereich von EPDM als elastomeres Dichtungsmaterial behandelt.

Ferner offenbart die WO 01/56613 A2 einen Erzeuger von sterilem Wasser, welcher auf Verlangen einen Strom von sterilem Wasser erzeugt. Ein Wassererhitzer erhitzt einströmendes, nicht-steriles Wasser auf eine ausreichende Temperatur, um eine Sterilisation des Wassers zu erreichen. Das sterile Wasser wird dann durch einen Wärmetauscher geführt, welcher überschüssige Hitze von dem sterilen Wasser auf das einströmende Wasser überträgt. Dies reduziert den Energieverbrauch des Wassererhitzers und reduziert die Temperatur des sterilen, gebrauchsbereiten Wassers. Um Kontaminierung des Wassers zu vermeiden, wird das sterile Wasser mittels eines sterilen Zuführwegs an eine Stelle geführt, an der das Wasser genutzt werden soll. Ein erster Teilbereich des Zuführwegs wird vor-sterilisiert durch fließenden Dampf oder heißes Wasser, welches durch den Wassererhitzer produziert wird, entlang des Zuführwegs. Ein zweiter Teilbereich des Zuführwegs wird sterilisiert durch eine antimikrobielle Flüssigkeit. Der erste und der zweite Teilbereich des Zuführwegs umfassen mindestens einen gemeinsamen Abschnitt.

Weiterhin beschreibt die EP 1 852 131 A1 einen abgeschlossenen Wasserspender umfassend ein Wasserspendesystem mit einem Kaltwasserreservoir zum Spenden von gekühltem Wasser von einem Auslauf und ein Heißwasserreservoir zum Spenden von heißem Wasser von einem Auslauf. Das Kaltwasserreservoir umfasst einen Zulauf, wobei der Zulauf lösbar verbindbar ist mit einer hängenden Flasche, um gespendetes Wasser zu ersetzen. Das System wird periodisch desinfiziert, um Bakterien in dem System zu töten, indem die Ausläufe zeitweise miteinander verbunden werden und in dem Heißwasserreservoir erzeugter Dampf oder erzeugtes heißes Wasser in Zirkulation gebracht wird.

US 4,547,339 A beschreibt ein Dampfsterilisationssystem für eine Abfüllmaschine mit Ausgabedüsen, die mit einer Produktzufuhrquelle verbunden sind, wobei das Sterilisationssystem eine neuartige Autoklavvorrichtung und ein neuartiges Kondensatablasssystem umfasst. Die Autoklavstruktur bewirkt, dass der Sterilisationsdampf durch das Innere der Ausgabedüse nach unten zu ihrem offenen unteren Ende strömt, wo er umkehrt und zwischen der Innenseite eines Umkehrrohrs und der Außenseite der Ausgabedüse nach oben zu einer Reihe von Löchern in der Seitenwand des Umkehrrohrs strömt, wo er austritt und zwischen der Außenseite des Umkehrrohrs und der Innenseite des Autoklavgehäuses nach unten strömt, um anschließend in eine Dampf- und Kondensatleitung zu gelangen. Das im Ausgaberohr gebildete Kondensat tropft aus dem Boden des Ausgaberohrs und durch eine Öffnung am Boden des Umkehrrohrs, wobei das Kondensat den Boden des Umkehrrohrs wirksam gegen Dampf abdichtet. Das Kondensatablasssystem umfasst ein Gegendruck- und Konstantablassventil, dessen Einlass mit der Dampf- und Kondensatleitung des Autoklaven verbunden ist, dessen Auslass mit einer Abflussleitung verbunden ist, und ein betätigbares Bypass-Ablassventil, das von einem internen Punkt im Ablassventil mit der Abflussleitung verbunden ist. Das Ablassventil wird steuerbar betätigt, um das Ablassventil wirksam zu entleeren und so die Rückstauung von Kondensat im System zu verhindern, ohne den Sterilisationsvorgang zu beeinträchtigen.

Eine technische Herausforderung bei der Bereitstellung von Flüssigkeiten wie insbesondere WFI besteht in der Regel darin, die Entnahmestelle, an welcher das WFI aus dem Leitungssystem entnommen wird, keimarm zu halten. Über die Entnahmestelle können ansonsten Keime und Verunreinigungen in das Leitungssystem und der Probenahme eindringen. In manchen Labors wird daher die Entnahmestelle, beispielsweise ein Hahn oder Stutzen, vor und/oder nach der Entnahme sterilisiert, beispielsweise mittels eines Brenners. Nachteilig an derartigen Verfahren ist jedoch, dass diese der Aufmerksamkeit des Bedienpersonals unterworfen sind. Nachlässigkeiten wirken sich unter Umständen auf das gesamte Leitungssystem aus. Zudem sind herkömmliche Techniken zur Sterilisierung der Entnahmestelle, wie beispielsweise die Verwendung eines Brenners, belastend für die Entnahmestelle und in der Regel schlecht automatisierbar.

### Aufgabe der Erfindung

Es ist daher Aufgabe der vorliegenden Erfindung, ein Leitungssystem sowie ein Verfahren bereitzustellen, welche die Nachteile bekannter Vorrichtungen und Verfahren zumindest weitgehend vermeiden. Insbesondere soll eine einfache, benutzerfreundliche und dennoch zuverlässige und sichere Entnahme mindestens einer keimarmen Flüssigkeit aus einem Leitungssystem vorgeschlagen werden, welche eine Verkeimung des Leitungssystems zumindest weitgehend vermeidet.

### Offenbarung der Erfindung

Diese Aufgabe wird gelöst durch ein Leitungssystemund ein Verfahren mit den Merkmalen der unabhängigen Patentansprüche. Vorteilhafte Weiterbildungen, welche einzeln oder in beliebiger Kombination realisierbar sind, sind in den abhängigen Ansprüchen dargestellt.

Die Erfindung wird durch die anhängigen Ansprüche definiert. Bevorzugte Ausführungsformend werden durch die anhängigen Ansprüche definiert.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, das das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nicht-optionale Merkmale, unangetastet bleiben.

In einem ersten Aspekt der vorliegenden Erfindung wird eine Entnahmevorrichtung zur Entnahme mindestens einer keimarmen Flüssigkeit aus einem Leitungssystem vorgeschlagen. Unter einer "Entnahmevorrichtung" ist dabei grundsätzlich eine beliebige Vorrichtung zu verstehen, welche es ermöglicht, eine Flüssigkeit aus einem Vorratsbehälter oder einer Leitung zu entnehmen.

Unter einer "keimarmen Flüssigkeit" ist allgemein im Rahmen der vorliegenden Erfindung eine Flüssigkeit zu verstehen, welche einem Keimreduktionsprozess unterworfen wurde. Die keimarme Flüssigkeit kann insbesondere eine Anzahl von weniger als 100 Keimen pro 100 ml aufweisen, insbesondere eine Anzahl von weniger als 50 Keimen pro 100 ml oder sogar eine Anzahl von weniger als 10 Keimen pro 100 ml. Insbesondere kann es sich bei der keimarmen Flüssigkeit um Wasser handeln, insbesondere Wasser der Qualität "WFI", "HPW" oder "AP" nach dem Europäischen Arzneibuch, beispielsweise nach der PH. Eur. 8. Ausgabe Band 3, Seiten 5248- 5257. Auch andere Flüssigkeiten, insbesondere nichtwasserhaltige Flüssigkeiten, können jedoch grundsätzlich eingesetzt werden.

Unter einem "Leitungssystem" ist im Rahmen der vorliegenden Erfindung eine Anordnung mit einer oder mehreren Leitungen und/oder einer oder mehreren Vorratseinrichtungen zu verstehen, durch welche eine keimarme Flüssigkeit geleitet werden kann oder in welchen eine keimarme Flüssigkeit bevorratet werden kann. Insbesondere kann das Leitungssystem ausgestaltet sein, um ein Eindringen von Keimen in eine in der Leitung oder der Vorratseinrichtung befindlichen Flüssigkeit zumindest weitgehend zu verhindern.

Die Entnahmevorrichtung umfasst mindestens einen Entnahmestutzen. Unter einem "Entnahmestutzen" ist im Rahmen der vorliegenden Erfindung ein Ende einer Rohrleitung zu verstehen, welches offen ist oder geöffnet werden kann. Insbesondere kann der Entnahmestutzen, wie nachfolgend noch näher beschrieben ist, ein Rohrstück umfassen, welches an einem Ende direkt oder indirekt mit dem Leitungssystem verbunden ist und an einem anderen Ende geöffnet ist oder geöffnet werden kann. Das Rohrstück kann beispielsweise über mindestens ein Ventil mit dem Leitungssystem verbunden sein, so dass ein freies Ausfließen der keimarmen Flüssigkeit aus dem Leitungssystem durch den Entnahmestutzen verhindert werden kann und nur nach Freigabe durch das Ventil erfolgen kann.

Die Entnahmevorrichtung umfasst weiterhin mindestens einen Koppelbogen. Der Koppelbogen ist mit dem Entnahmestutzen lösbar verbunden. Mindestens ein Rohrende des Entnahmestutzens ragt in einen Innenraum des Koppelbogens hinein. Das in den Entnahmestutzen hineinragende Rohrende des Entnahmestutzens ist in dem Koppelbogen auf seiner Innenseite, seiner Außenseite und seiner Stirnseite mit Reindampf, beispielsweise "Pure Steam", Sterilisierdampf oder Pharmazeutischen Reinstdampf, sterilisierbar.

Unter einem "Koppelbogen" ist allgemein im Rahmen der vorliegenden Erfindung eine Abdeckung zu verstehen, welche einen Teil des Entnahmestutzens, im vorliegenden Fall insbesondere das Rohrende, aufnehmen kann und dieses nach außen abschließen kann. Der Koppelbogen kann insbesondere mindestens einen Hohlraum aufweisen, in welchen das Rohrende hineinragen kann, um dort von Reindampf umspült zu werden und desinfiziert zu werden. Der Hohlraum kann insbesondere derart dimensioniert sein, dass die Außenseite, die Stirnseite und die Innenseite des Rohrendes frei liegen und frei für den Dampf zugänglich sind. Wie nachfolgend noch näher ausgeführt wird, kann der Koppelbogen insbesondere ebenfalls zumindest teilweise rohrförmig ausgestaltet sein, mit einem Innendurchmesser, welcher größer ist als das Rohrende das Entnahmestutzens, so dass das Rohrende in den rohrförmigen Koppelbogen eingeführt werden kann. Das Rohr des Koppelbogens kann gerade oder vorzugsweise auch gekrümmt ausgestaltet sein. Der Koppelbogen kann ebenfalls einen Rohrstutzen aufweisen, mit einem Ende, welches auf das Rohrende des Entnahmestutzens aufgeschoben werden kann. Auch andere Ausgestaltungen sind jedoch grundsätzlich möglich.

Unter einer "lösbaren Verbindung" ist allgemein eine Verbindung zu verstehen, welche reversibel ausgestaltet ist. Insbesondere kann es sich hierbei um eine kraftschlüssige Verbindung zwischen dem Entnahmestutzen und dem Koppelbogen handeln. Diese kraftschlüssige Verbindung kann beispielsweise mindestens eine Schraubverbindung umfassen. Zur Entnahme der Flüssigkeit kann beispielsweise der Koppelbogen von dem Entnahmestutzen entfernt werden, so dass die keimarme Flüssigkeit aus dem Entnahmestutzen ausfließen kann, sobald ein Ventil geöffnet ist. Im sonstigen Zustand, wenn keine keimarme Flüssigkeit entnommen werden soll, kann der Koppelbogen auf das Rohrende des Entnahmestutzens aufgesetzt sein, so dass das Rohrende durch den Koppelbogen gegen Verkeimung geschützt ist.

Unter einer Anordnung, bei welcher das Rohrende des Entnahmestutzens in dem Koppelbogen auf seiner Innenseite, seiner Außenseite und seiner Stirnseite mit Reindampf sterilisierbar ist, ist eine Anordnung zu verstehen, bei welcher das Rohrende derart in dem Koppelbogen frei aufgenommen ist, dass die Innenseite, die Außenseite und die Stirnseite desselben für Reindampf, welcher durch den Entnahmestutzen selbst und/oder durch den Koppelbogen zu dem Rohrende geleitet wird, frei zu der Innenseite, der Außenseite und der Stirnseite gelangen kann, um dort Keime abzutöten.

Insgesamt lässt sich auf diese Weise einerseits, wenn keine keimarme Flüssigkeit entnommen wird, eine Verkeimung des Entnahmestutzens mittels des Koppelbogens wirksam verhindern. Vor oder auch nach einer Entnahme der keimarmen Flüssigkeit kann das Rohrende des Koppelbogens mit Reindampf sterilisiert werden, ohne dass Bedienpersonal mit dem Reindampf in Kontakt gerät. Dieses Verfahren lässt sich auch automatisieren.

Das Rohrende des Entnahmestutzens kann insbesondere ringförmig von einer Innenwand des Koppelbogens umschlossen sein, insbesondere wenn der Koppelbogen selbst rohrförmig ausgestaltet ist. Dabei kann das Rohrende von der Innenwand des Koppelbogens beabstandet aufgenommen sein, so dass sich ein Ringspalt zwischen der Innenwand und dem Rohrende ausbildet, in welchen der Reindampf eindringen kann. Der Ringspalt kann insbesondere eine Spaltdicke von 0.2 mm bis 5 mm, vorzugsweise von 0.5 mm bis 3 mm und besonders bevorzugt von 0.7 mm bis 1 mm aufweisen. Auch andere Dimensionen sind jedoch grundsätzlich möglich.

Wie oben ausgeführt, kann der Entnahmestutzen insbesondere rohrförmig ausgestaltet sein, beispielsweise zumindest abschnittsweise zylindrisch mit einem kreisförmigen, ovalen oder polygonalen Querschnitt, und kann beispielsweise einen Innendurchmesser aufweisen, gegebenenfalls auch einen Äquivalent-Innendurchmesser. Der Innendurchmesser kann insbesondere 3 mm bis 40 mm, insbesondere 10 mm bis 20 mm, betragen. Auch andere Dimensionen sind jedoch grundsätzlich möglich.

Wie ebenfalls oben bereits ausgeführt, kann insbesondere auch der Koppelbogen rohrförmig ausgestaltet sein und einen Innendurchmesser aufweisen. Der Innendurchmesser des Koppelbogens kann insbesondere 4 mm bis 50 mm, insbesondere 11 mm bis 42 mm, betragen. Auch andere Dimensionen sind jedoch grundsätzlich möglich. Insbesondere kann der Innendurchmesser des Koppelbogens zumindest in einem Bereich, in welchen das Rohrende des Entnahmestutzens hineinragt, einen größeren Innendurchmesser aufweisen als der Entnahmestutzen.

Da vorzugsweise eine Sterilisation des Rohrendes mit Reindampf in dem Koppelbogen erfolgt, ist es vorteilhaft, wenn der Koppelbogen zumindest abschnittsweise thermisch isoliert ausgestaltet ist, damit Bedienpersonal beim Abnehmen des Koppelbogens von dem Entnahmestutzen nicht verletzt wird. Dementsprechend kann der Koppelbogen zumindest teilweise doppelwandig ausgestaltet sein. Alternativ oder zusätzlich zu einer doppelwandigen Ausgestaltung sind jedoch auch andere thermische Isolierungen denkbar. Eine doppelwandige Ausgestaltung ist jedoch leicht realisierbar und insbesondere leicht zu reinigen. Die doppelwandige Ausgestaltung kann insbesondere derart sein, dass ein Hohlraum zwischen zwei Wänden des Koppelbogens vorgesehen ist, welcher vorzugsweise vollständig abgeschlossen ist, so dass keine Keime in diesen Hohlraum eindringen können.

Der Koppelbogen kann insbesondere zumindest in einem Bereich, in welchen das Rohrende des Entnahmestutzens hineinragt, doppelwandig ausgestaltet sein. Dabei kann der Koppelbogen in dem gesamten Bereich, in welchen das Rohrende des Entnahmestutzens hineinragt, doppelwandig ausgestaltet sein oder lediglich einem Teil dieses Bereichs.

Die doppelwandige Ausgestaltung kann insbesondere dadurch erzeugt sein, dass mindestens eine Außenhülle mit einem gegenüber einem Außendurchmesser des Koppelbogens vergrößerten Innendurchmesser auf den Koppelbogen aufgeschoben ist. Der Koppelbogen kann also ganz oder teilweise als Doppelrohr ausgestaltet sein, wobei sich zwischen den Rohren ein isolierender Ringspalt ausbilden kann. Die Außenhülle, also das Außenrohr, kann dabei mit dem Koppelbogen, insbesondere einem Innenrohr, verschweißt sein.

Das Rohrende des Entnahmestutzens kann insbesondere um mindestens 10 mm, vorzugsweise um mindestens 20 mm, in den Innenraum des Koppelbogens hineinragen. Das Rohrende des Entnahmestutzens kann insbesondere um mindestens 10 mm bis 50 mm in den Innenraum des Koppelbogens hineinragen.

Der Entnahmestutzen kann insbesondere L-förmig ausgestaltet sein. Auch der Koppelbogen kann L-förmig ausgestaltet ist. Dabei kann ein Ende des L des Entnahmestutzens beispielsweise direkt oder indirekt mit dem Leitungssystem verbunden sein, und das andere Ende des L des Entnahmestutzens kann mit dem Koppelbogen verbunden sein. Dementsprechend kann ein Ende des L des Koppelbogens mit dem Entnahmestutzen verbunden sein. Ein anderes Ende des L des Koppelbogens kann beispielsweise direkt oder indirekt mit einem Abfluss verbunden sein. Die Entnahmevorrichtung kann somit insgesamt, mit dem Entnahmestutzen und dem Koppelbogen in verbundenem Zustand, U-förmig ausgestaltet sein, mit einem eckigen oder abgerundeten U. Auf diese Weise lässt sich beispielsweise die Entnahmevorrichtung mit den beiden Enden des U an einem Gehäuse oder an einer Wand befestigen.

Eine Stirnseite des Rohrendes des Entnahmestutzens kann insbesondere schräg gegenüber einer Rohrachse des Rohrendes verlaufen. Dieses abgeschrägte Rohrende kann ein Abtropfen von keimarmer Flüssigkeit begünstigen, so dass beispielsweise keine Flüssigkeitstropfen an der Spitze des Rohrendes verbleiben. Insbesondere kann die Entnahmevorrichtung derart montiert werden, dass die Spitze des Rohrendes nach unten weist. Ein geöffnetes Ende des Koppelbogens, welches das Rohrende aufnimmt, kann dementsprechend nach oben weisen.

Der Koppelbogen kann insbesondere als Rohrstück ausgestaltet sein, wobei eine erste Öffnung des Rohrstücks das Rohrende des Entnahmestutzens aufnimmt und wobei eine zweite Öffnung des Rohrstücks mit Reindampf beaufschlagbar ist, so dass Reindampf durch das Rohrstück von der zweiten Öffnung zu der ersten Öffnung gelangen kann und dort das Rohrende des Entnahmestutzens sterilisieren kann. Das Rohrstück kann insbesondere L-förmig gebogen sein, mit einem eckigen oder abgerundeten L.

Der Koppelbogen kann insbesondere über mindestens eine kraftschlüssige und/oder formschlüssige Verbindung mit dem Entnahmestutzen verbunden sein. Insbesondere kann der Koppelbogen mit dem Entnahmestutzen über mindestens eine Schraubverbindung lösbar verbunden sein. Beispielsweise kann die Schraubverbindung mindestens eine Kombination umfassen, ausgewählt aus der Gruppe bestehend aus: einem an dem Koppelbogen fixierten Außengewinde und einer an dem Entnahmestutzen angeordneten Überwurfmutter; einem an dem Entnahmestutzen angeordnetem Außengewinde und einer an dem Koppelbogen angeordneten Überwurfmutter. Das Teil der Kombination der Entnahmevorrichtung, welches die Überwurfmutter trägt, kann beispielsweise einen Kragen oder einen Flansch aufweisen, an welchem die Überwurfmutter angreift, wenn diese mit dem Außengewinde des jeweiligen anderen Teils verschraubt wird.

Die Entnahmevorrichtung kann insbesondere ganz oder teilweise, insbesondere an dem Rohrende, aus Stahl, insbesondere aus Edelstahl, hergestellt sein. Stahl und insbesondere Edelstahl sind besonders einfach zu reinigen und zu desinfizieren und verhindern weitgehend, dass eine Verkeimung auftreten kann.

Die Entnahmevorrichtung kann insbesondere ganz oder teilweise, insbesondere an dem Rohrende, mit einer möglichst geringen Oberflächenrauigkeit ausgestaltet sein, um möglichst wenig Angriffsfläche für eine Verkeimung zu bieten. So ist es vorteilhaft, wenn zumindest das Rohrende des Entnahmestutzens eine Oberflächenrauigkeit RMS von 0.1 bis 2.0 Mikrometern, insbesondere von 0.2 bis 0.6 Mikrometern und besonders bevorzugt von 0.4 Mikrometern oder weniger aufweist.

Die Entnahmevorrichtung kann weiterhin mindestens einen mit dem Koppelbogen verbundenen elektrischen Kontakt zur Verbindung mit einer Steuerung und zur Erkennung eines Zustandes, in welchem der Koppelbogen mit dem Entnahmestutzen verbunden ist, aufweisen. So kann beispielsweise der elektrische Kontakt starr mit dem Koppelbogen verbunden sein, wobei eine Anordnung der Entnahmevorrichtung relativ zu der Steuerung derart erfolgen kann, dass nur in einem Zustand, wenn der Koppelbogen mit dem Entnahmestutzen verbunden ist, der elektrische Kontakt mit der Steuerung verbunden ist. Auf diese Weise kann die Steuerung beispielsweise verhindern, dass Reindampf in die Entnahmevorrichtung geleitet wird, wenn der Entnahmestutzen nicht auf das Rohrende aufgesetzt ist. So kann verhindert werden, dass Bedienpersonal durch Reindampf verletzt wird.

In einem weiteren Aspekt der vorliegenden Erfindung wird ein Leitungssystem zur Bereitstellung mindestens einer keimarmen Flüssigkeit vorgeschlagen. Das Leitungssystem umfasst mindestens ein Rohrleitungssystem für die keimarme Flüssigkeit. Unter einem "Rohrleitungssystem" ist dabei allgemein eine Anordnung mit einer oder mehreren Rohrleitungen und/oder einer oder mehreren Vorratseinrichtungen zu verstehen, durch welche eine oder mehrere Flüssigkeiten geleitet und/oder in welchen eine oder mehrere Flüssigkeiten gelagert werden können. Das Leitungssystem umfasst weiterhin mindestens eine erfindungsgemäße Entnahmevorrichtung, beispielsweise gemäß einer oder mehrerer der oben beschriebenen Ausgestaltungen und/oder gemäß einer oder mehrerer der nachfolgend noch näher beschriebenen Ausgestaltungen. Die Entnahmevorrichtung ist mit dem Rohrleitungssystem verbunden, insbesondere dergestalt, dass eine fluidische Verbindung zwischen dem Rohrleitungssystem und der Entnahmevorrichtung besteht und/oder dass die Entnahmevorrichtung in das Rohrleitungssystem eingebunden ist. Das Leitungssystem kann eine oder auch mehrere Entnahmevorrichtungen umfassen, so dass an einer oder mehreren Entnahmestellen keimarme Flüssigkeit aus dem Rohrleitungssystem entnommen werden kann.

Das Leitungssystem weist weiterhin mindestens einen Behälter zur Aufnahme der keimarmen Flüssigkeit auf. Der Behälter ist mit dem Rohrleitungssystem verbunden. Der Behälter kann beispielsweise derart ausgestaltet sein, dass keimarme Flüssigkeit dauerhaft durch diesen Behälter gepumpt wird. Beispielsweise kann in dem Behälter an einem oberen Ende mindestens eine Zufuhr, beispielsweise mindestens eine Sprühdüse, angeordnet sein, welche keimarme Flüssigkeit in den Behälter befördert, und an einem unteren Ende ein Ablauf, durch welchen keimarme Flüssigkeit aus dem Behälter entnommen wird. So kann beispielsweise die keimarme Flüssigkeit im Kreislauf durch das Rohrleitungssystem gepumpt werden, unter Einbeziehung des Behälters. Das Rohrleitungssystem ist ringförmig ausgestaltet und weist mindestens eine Pumpe auf, welche eingerichtet ist, um die keimarme Flüssigkeit in einem Kreislauf durch das Rohrleitungssystem und den Behälter zu pumpen.

Wie oben ausgeführt, kann die mindestens eine Entnahmevorrichtung fluidisch mit dem Rohrleitungssystem verbunden sein, beispielsweise indem die Entnahmevorrichtung unmittelbar in das Rohrleitungssystem eingebunden ist oder mittels einer fluidischen Verbindung mit dem Rohrleitungssystem verbunden ist. Das Rohrleitungssystem weist mindestens einen Abzweig auf. Der Abzweig ist mit der Entnahmevorrichtung verbunden. Der Abzweig kann beispielsweise mindestens ein Rohrstück umfassen, welches an einem Ende direkt oder indirekt mit dem Rohrleitungssystem verbunden ist, beispielsweise über ein T-Stück oder ein Mehrwegeventil, und welches an einem anderen Ende direkt oder indirekt mit der Entnahmevorrichtung verbunden ist. Insbesondere kann zwischen dem Abzweig und der Entnahmevorrichtung mindestens ein Ventil vorgesehen sein. Hierbei kann es sich um ein 2-Wege-Ventil oder auch um ein Mehrwegeventil handeln oder auch beispielsweise einfach um ein Absperrventil.

Wie oben ausgeführt, kann das Leitungssystem neben dem Rohrleitungssystem eine oder mehrere weitere Komponenten umfassen, welche fluidisch oder auch nicht-fluidisch sein können. Insbesondere können auch eine oder mehrere mechanische Komponenten oder auch elektronische Komponenten umfasst sein. So kann das Leitungssystem insbesondere weiterhin mindestens eine Steuerung aufweisen, welche eine oder mehrere Steuerungs- und/oder Regelungsaufgaben übernehmen kann. Insbesondere kann die Steuerung über mindestens einen elektrischen Kontakt mit dem Koppelbogen verbindbar sein und kann eingerichtet sein, um zu erkennen, ob der Koppelbogen mit dem Entnahmestutzen verbunden ist. Wie oben beschrieben, kann dementsprechend die Steuerung insbesondere weiterhin eingerichtet sein, um nur dann Reindampf bereitzustellen, wenn der Koppelbogen mit dem Entnahmestutzen verbunden ist, und andernfalls eine Bereitstellung von Reindampf zu verhindern. Beispielsweise kann die Steuerung ein oder mehrere Ventile ansteuern, welche eine Beaufschlagung des Rohrendes des Entnahmestutzens mit dem Reindampf freigeben können. Wie oben ausgeführt, kann dadurch mittels der Steuerung beispielsweise verhindert werden, dass Bedienpersonal durch Reindampf verletzt wird, wenn der Koppelbogen von dem Entnahmestutzen entfernt ist.

Zwischen dem Abzweig und der Entnahmevorrichtung ist mindestens eine Zufuhr für Reindampf angeordnet, so dass Reindampf von der Zufuhr durch den Entnahmestutzen in den Koppelbogen leitbar ist, um dort das Rohrende des Entnahmestutzens zu sterilisieren. Die Zufuhr für den Reindampf kann insbesondere mit mindestens einem Ventil verschließbar sein. Alternativ oder zusätzlich kann eine Zufuhr für Reindampf jedoch auch an mindestens einer weiteren Stelle angeordnet sein. So kann beispielsweise mindestens eine Zufuhr für Reindampf zwischen einem Abfluss und dem Koppelbogen vorgesehen sein.

Der Koppelbogen kann insbesondere an einem dem Entnahmestutzen abgewandten Ende mit mindestens einem Abfluss verbunden sein. Zwischen dem Koppelbogen und dem Abfluss kann insbesondere mindestens ein Ventil vorgesehen sein. Wie oben ausgeführt, kann weiterhin mindestens eine Zufuhr für Reindampf zwischen dem Koppelbogen und dem Abfluss angeordnet sein. Weiterhin kann zwischen dem Koppelbogen und dem Abfluss, insbesondere zwischen dem mindestens einen Ventil und dem Abfluss, mindestens ein Kondensatableiter vorgesehen sein.

In einem weiteren Aspekt der vorliegenden Erfindung wird ein Verfahren zur Entnahme mindestens einer keimarmen Flüssigkeit aus einem Leitungssystem vorgeschlagen. Das Verfahren verwendet das Leitungssystem gemäß der vorliegenden Erfindung, so dass bezüglich möglicher Ausgestaltungen auf die obige Beschreibung sowie auch auf die nachfolgende Beschreibung von Ausführungsbeispielen verwiesen werden kann. Das Verfahren umfasst die folgenden Schritte, welche vorzugsweise in der genannten Reihenfolge durchgeführt werden. Auch eine andere Reihenfolge ist jedoch grundsätzlich möglich sowie ein Verfahren, bei welchem ein oder mehrere der Verfahrensschritte zeitlich überlappend, zeitgleich oder auch einfach oder mehrfach wiederholt durchgeführt werden. Das Verfahren kann zusätzliche, nicht genannte Schritte umfassen.

Das Verfahren umfasst die folgenden Schritte:
a) Bereitstellen eines Leitungssystems nach einem der vorhergehenden, ein Leitungssystem betreffenden Ansprüche;
b) Einleiten von Reindampf in den Koppelbogen und Sterilisieren des Rohrendes des Entnahmestutzen in dem Innenraum des Koppelbogens durch Umspülen des Rohrendes mit dem Reindampf;
c) Lösen und Entfernen des Koppelbogens von dem Entnahmestutzen; und
d) Entnahme der keimarmen Flüssigkeit aus dem Entnahmestutzen.

Unter "Reindampf" ist im Rahmen der vorliegenden Erfindung grundsätzlich beliebiger Dampf zu verstehen, insbesondere Wasserdampf. Der Dampf kann insbesondere eine definierte Qualität aufweisen. Beispielsweise kann es sich bei dem Reindampf um Dampf handeln, welcher gefiltert ist, beispielsweise mit einer Filterporengröße ≤10 Mikrometer oder ≤4 Mikrometer. Alternativ oder zusätzlich kann es sich bei dem Reindampf um Dampf der keimarmen Flüssigkeit selbst handeln, also die keimarme Flüssigkeit in gasförmigem Zustand, beispielsweise Wasser der Qualität "WFI", "HPW" oder "AP" in Dampfform. Bei dem Reindampf kann es sich insbesondere um eine oder mehrere der folgenden Dampfarten handeln: "Pure Steam" (definiert z.B. in U.S. Pharmacopeia); Sterilisierdampf (definiert z.B. in DIN 58950 Teil 7); Pharmazeutischen Reinstdampf (definiert z.B. in DIN 58950 Teil 7). Der Dampf kann insbesondere eine höhere Temperatur als die Siedetemperatur der zu Grunde liegenden Flüssigkeit aufweisen. Insbesondere kann es sich um Dampf, beispielsweise Wasserdampf, handeln, welcher eine Temperatur von >100 °C aufweist, insbesondere eine Temperatur von ≥121 °C. Beispielsweise kann der Reindampf Temperaturen von 121 °C bis ca. 140 °C aufweisen.

In einem weiteren Aspekt der vorliegenden Erfindung wird eine Verwendung einer Entnahmevorrichtung gemäß der vorliegenden Erfindung zur Entnahme mindestens einer keimarmen Flüssigkeit aus einem Leitungssystem vorgeschlagen. Die keimarme Flüssigkeit kann insbesondere Wasser für Injektionszwecke sein oder umfassen.

Die vorgeschlagenen Vorrichtungen und das vorgeschlagene Verfahren weisen gegenüber bekannten Vorrichtungen und Verfahren zahlreiche Vorteile auf. Insbesondere lässt sich die oben skizzierte Problematik, dass das Rohrende einer Entnahmevorrichtung bei einer Entnahme keimarmer Flüssigkeit der Gefahr der Verkeimung ausgesetzt ist, auf einfache und dennoch zuverlässige Weise lösen. Eine manuelle Erhitzung des Rohrendes, beispielsweise durch einen Benutzer mit einem Brenner, lässt sich vermeiden. Insbesondere lässt sich durch die Verwendung von Reindampf, in Kombination mit der allseitigen Sterilisierung des Rohrendes durch denselben, eine effiziente Keimabtötung an dem Rohrende vor oder auch nach einer Entnahme bewerkstelligen, welche auch automatisierbar ist. Dementsprechend kann das gesamte Verfahren beispielsweise durch eine Steuerung gesteuert werden. Der Einfluss menschlicher Tätigkeit und insbesondere Nachlässigkeit, welcher ansonsten gegebenenfalls zu einer Verkeimung des gesamten Leitungssystems, insbesondere des gesamten Reinstwasser-Leitungssystems, führen könnte, lässt sich hierdurch wirksam reduzieren. Gleichzeitig lässt sich durch die Verwendung des Koppelbogens eine Gefährdung des Bedienpersonals durch den Reindampf verhindern. Es sei darauf hingewiesen, dass, alternativ oder zusätzlich zu Reindampf, auch andere sterilisierende Medien verwendet werden können, beispielsweise andere sterilisierende Gase. Diese können beispielsweise durch den Koppelbogen abgesaugt werden, bevor der Koppelbogen entfernt wird.

Zusammenfassend sind im Rahmen der vorliegenden Erfindung folgende Ausführungsformen und Beispiele besonders bevorzugt:
Es wird eine Entnahmevorrichtung zur Entnahme mindestens einer keimarmen Flüssigkeit aus einem Leitungssystem beschrieben, umfassend mindestens einen Entnahmestutzen, weiterhin umfassend mindestens einen Koppelbogen, wobei der Koppelbogen mit dem Entnahmestutzen lösbar verbunden ist, wobei mindestens ein Rohrende des Entnahmestutzens in einen Innenraum des Koppelbogens hineinragt und in dem Koppelbogen auf seiner Innenseite, seiner Außenseite und seiner Stirnseite mit Reindampf sterilisierbar ist.

Das Rohrende des Entnahmestutzens kann ringförmig von einer Innenwand des Koppelbogens umschlossen sein, wobei das Rohrende von der Innenwand beabstandet ist, so dass sich ein Ringspalt zwischen der Innenwand und dem Rohrende ausbildet, in welchen der Reindampf eindringen kann.

Der Ringspalt kann eine Spaltdicke von 0.2 mm bis 5 mm, vorzugsweise von 0.5 mm bis 3 mm und besonders bevorzugt von 0.7 mm bis 1 mm aufweisen.

Der Entnahmestutzen kann rohrförmig ausgestaltet sein und kann einen Innendurchmesser aufweisen.

Der Innendurchmesser kann 3 mm bis 40 mm, insbesondere 10 mm bis 20 mm, betragen.

Der Koppelbogen kann rohrförmig ausgestaltet sein und einen Innendurchmesser aufweisen.

Der Innendurchmesser des Koppelbogens kann 4 mm bis 50 mm, insbesondere 11 mm bis 42 mm, betragen.

Der Innendurchmesser des Koppelbogens kann zumindest in einem Bereich, in welchen das Rohrende des Entnahmestutzens hineinragt, einen größeren Innendurchmesser aufweisen als der Entnahmestutzen.

Der Koppelbogen kann zumindest teilweise doppelwandig ausgestaltet sein.

Der Koppelbogen kann zumindest in einem Bereich, in welchen das Rohrende des Entnahmestutzens hineinragt, doppelwandig ausgestaltet sein.

Die doppelwandige Ausgestaltung kann dadurch erzeugt sein, dass mindestens eine Außenhülle mit einem gegenüber einem Außendurchmesser des Koppelbogens vergrößerten Innendurchmesser auf den Koppelbogen aufgeschoben ist.

Die Außenhülle kann mit dem Koppelbogen verschweißt sein.

Das Rohrende des Entnahmestutzens kann um mindestens 10 mm, vorzugsweise um mindestens 20 mm, in den Innenraum des Koppelbogens hineinragen.

Das Rohrende des Entnahmestutzens kann um mindestens 10 mm bis 50 mm in den Innenraum des Koppelbogens hineinragen.

Der Entnahmestutzen kann L-förmig ausgestaltet sein und der Koppelbogen kann L-förmig ausgestaltet sein, so dass die Entnahmevorrichtung insgesamt, mit dem Entnahmestutzen und dem Koppelbogen in verbundenem Zustand, U-förmig ausgestaltet ist.

Die Stirnseite des Rohrendes des Entnahmestutzens kann schräg gegenüber einer Rohrachse des Rohrendes verlaufen.

Der Koppelbogen kann als Rohrstück ausgestaltet sein. Eine erste Öffnung des Rohrstücks kann das Rohrende des Entnahmestutzens aufnehmen und eine zweite Öffnung des Rohrstücks kann mit Reindampf beaufschlagbar sein, so dass Reindampf durch das Rohrstück von der zweiten Öffnung zu der ersten Öffnung gelangen kann und dort das Rohrende des Entnahmestutzens sterilisieren kann.

Das Rohrstück kann L-förmig gebogen sein.

Der Koppelbogen kann mit dem Entnahmestutzen über eine Schraubverbindung lösbar verbunden sein.

Die Schraubverbindung kann mindestens eine Kombination umfassen, ausgewählt aus der Gruppe bestehend aus: einem an dem Koppelbogen fixierten Außengewinde und einer an dem Entnahmestutzen angeordneten Überwurfmutter; einem an dem Entnahmestutzen angeordnetem Außengewinde und einer an dem Koppelbogen angeordneten Überwurfmutter.

Die Entnahmevorrichtung kann ganz oder teilweise aus Stahl, insbesondere aus Edelstahl, hergestellt sein.

Das Rohrende des Entnahmestutzens kann eine Oberflächenrauigkeit RMS von 0.1 bis 2.0 Mikrometern, insbesondere von 0.2 bis 0.6 Mikrometern und besonders bevorzugt von 0.4 Mikrometern aufweisen.

Die Entnahmevorrichtung kann weiterhin einen mit dem Koppelbogen verbundenen elektrischen Kontakt zur Verbindung mit einer Steuerung und zur Erkennung eines Zustandes aufweisen, in welchem der Koppelbogen mit dem Entnahmestutzen verbunden ist.

Es wird ein Leitungssystem zur Bereitstellung mindestens einer keimarmen Flüssigkeit beschrieben, umfassend mindestens ein Rohrleitungssystem für die keimarme Flüssigkeit, weiterhin umfassend mindestens eine Entnahmevorrichtung wie sie bereits beschrieben wurde, wobei die Entnahmevorrichtung mit dem Rohrleitungssystem verbunden ist.

Das Leitungssystem weist weiterhin mindestens einen Behälter zur Aufnahme der keimarmen Flüssigkeit auf. Der Behälter ist mit dem Rohrleitungssystem verbunden.

Das Rohrleitungssystem ist ringförmig ausgestaltet und weist mindestens eine Pumpe auf. Die Pumpe ist eingerichtet, um die keimarme Flüssigkeit in einem Kreislauf durch das Rohrleitungssystem und den Behälter zu pumpen.

Das Rohrleitungssystem weist mindestens einen Abzweig auf, welcher mit der Entnahmevorrichtung verbunden ist.

Zwischen dem Abzweig und der Entnahmevorrichtung kann mindestens ein Ventil vorgesehen sein.

Zwischen dem Abzweig und der Entnahmevorrichtung ist mindestens eine Zufuhr für Reindampf angeordnet, so dass Reindampf von der Zufuhr durch den Entnahmestutzen in den Koppelbogen leitbar ist, um dort das Rohrende des Entnahmestutzens zu sterilisieren.

Die Zufuhr für den Reindampf kann mit mindestens einem Ventil verschließbar sein.

Das Leitungssystem kann weiterhin mindestens eine Steuerung aufweisen.Die Steuerung kann über mindestens einen elektrischen Kontakt mit dem Koppelbogen verbindbar sein und eingerichtet sein, um zu erkennen, ob der Koppelbogen mit dem Entnahmestutzen verbunden ist.Die Steuerung kann weiterhin eingerichtet sein, um nur dann Reindampf bereitzustellen, wenn der Koppelbogen mit dem Entnahmestutzen verbunden ist, und andernfalls eine Bereitstellung von Reindampf zu verhindern.

Der Koppelbogen kann an einem dem Entnahmestutzen abgewandten Ende mit mindestens einem Abfluss verbunden sein.

Zwischen dem Koppelbogen und dem Abfluss kann mindestens ein Ventil vorgesehen sein.

Zwischen dem Koppelbogen und dem Abfluss, insbesondere zwischen dem mindestens einen Ventil und dem Abfluss, kann mindestens ein Kondensatableiter vorgesehen sein.

Es wird ein Verfahren zur Entnahme mindestens einer keimarmen Flüssigkeit aus einem Leitungssystem beschrieben, insbesondere einem Reinstwasser-Leitungssystem, umfassend die folgenden Schritte:
a) Bereitstellen eines Leitungssystems wie es bereits beschrieben wurde;
b) Einleiten von Reindampf in den Koppelbogen und Sterilisieren des Rohrendes des Entnahmestutzen in dem Innenraum des Koppelbogens durch Umspülen des Rohrendes mit dem Reindampf;
c) Lösen und Entfernen des Koppelbogens von dem Entnahmestutzen; und
d) Entnahme der keimarmen Flüssigkeit aus dem Entnahmestutzen.

Der Reindampf kann eine Temperatur von 121 °C bis 140 °C aufweisen.

Es wird eine Verwendung einer Entnahmevorrichtung wie sie bereits beschrieben wurde offenbart zur Entnahme mindestens einer keimarmen Flüssigkeit aus einem Leitungssystem, insbesondere einem Reinstwasser-Leitungssystem.

Die keimarme Flüssigkeit kann Wasser für Injektionszwecke umfassen.

### Kurze Beschreibung der Figuren

Weitere Einzelheiten und optionale Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:
- Figur 1: ein Schema eines Ausführungsbeispiels eines Leitungssystems mit einer Entnahmevorrichtung;
- Figur 2: ein Ausführungsbeispiel einer Entnahmevorrichtung;
- Figur 3: einen Entnahmestutzen der Entnahmevorrichtung gemäß Figur 2; und
- Figur 4: eine perspektivische Darstellung der Entnahmevorrichtung gemäß Figur 2, montiert an einer Wand.

### Beschreibung der Ausführungsbeispiele

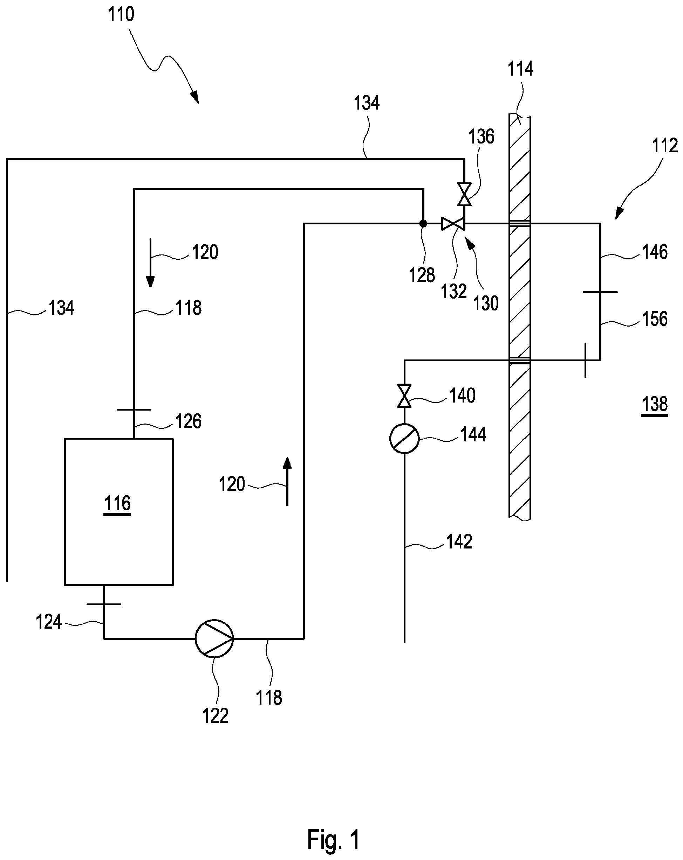

In den Figuren 1 bis 4 sind Beispiele eines Leitungssystems 110 zur Bereitstellung mindestens einer keimarmen Flüssigkeit und einer Entnahmevorrichtung 112 zur Entnahme mindestens einer keimarmen Flüssigkeit aus dem Leitungssystem 110 dargestellt. Bei der keimarmen Flüssigkeit kann es sich insbesondere um Water for Injection, also Wasser für Injektionszwecke, handeln. Dementsprechend wird nachfolgend, wenn von der keimarmen Flüssigkeit die Rede ist, auch allgemein die Bezeichnung WFI verwendet.

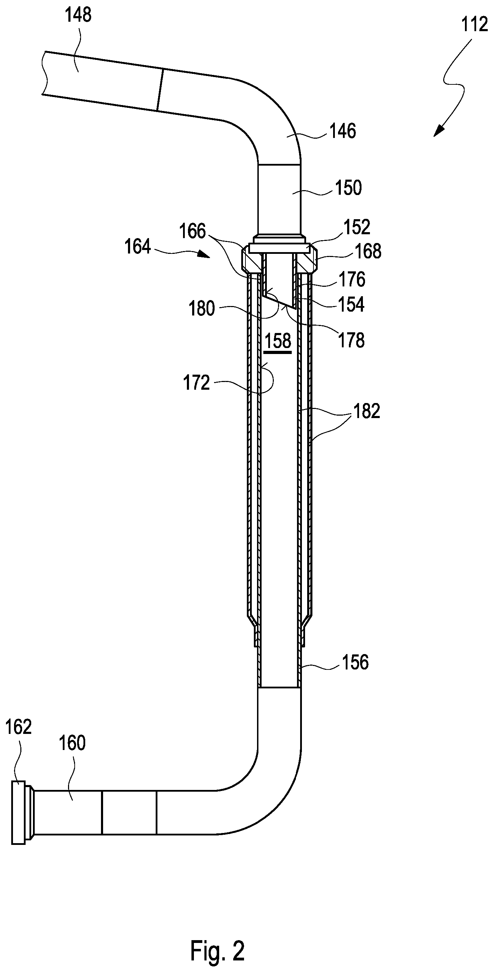

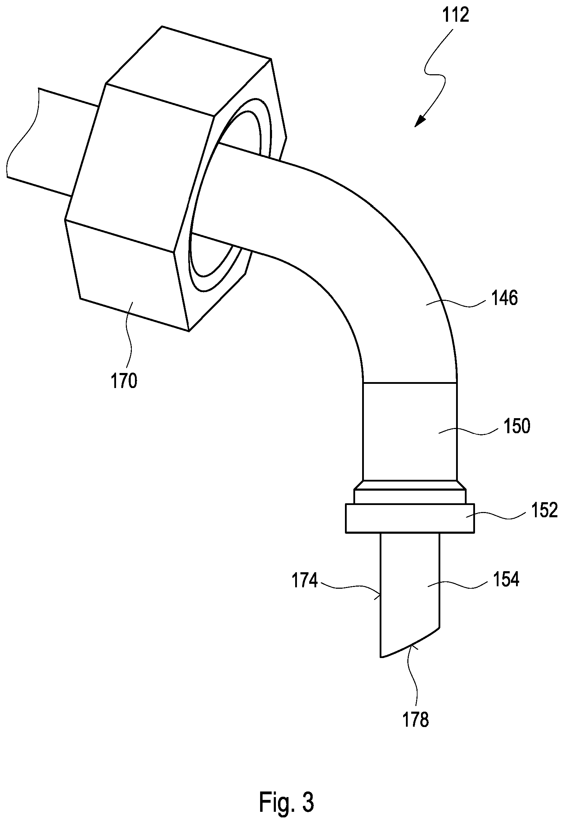

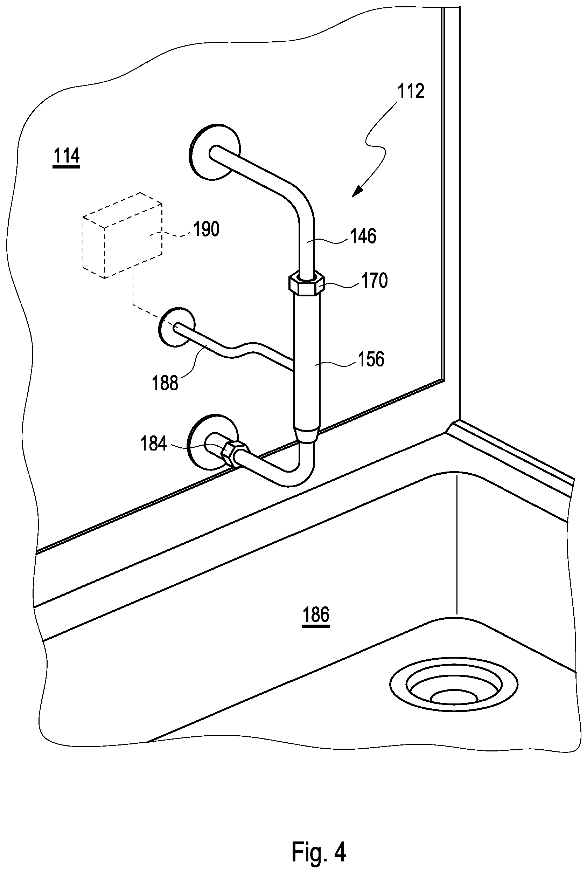

In Figur 1 ist ein Schema des Leitungssystems 110 in einer möglichen Ausgestaltung dargestellt. Die Figuren 2 bis 4 zeigen dann Einzelheiten der Entnahmevorrichtung 112 in einer Schnittdarstellung (Figur 2), in einer perspektivischen Darstellung mit entferntem Koppelbogen (Figur 3) und in einer perspektivischen Darstellung in einer Montage an einer Wand 114 (Figur 4). Die Figuren werden im Folgenden gemeinsam erläutert.

Das Leitungssystem 110 in der Darstellung gemäß Figur 1 umfasst einen Behälter 116 zur Aufnahme der keimarmen Flüssigkeit, im Zusammenhang mit WFI auch einfach als WFI-Behälter zu bezeichnen. Dieser Behälter 116 wird über eine nicht näher dargestellte Zuleitung, beispielsweise aus einer Aufbereitungsvorrichtung, mit der keimarmen Flüssigkeit befüllt.

Der Behälter 116 ist in ein Rohrleitungssystem 118 eingebunden. In diesem Rohrleitungssystem kann die keimarme Flüssigkeit insbesondere in einem Kreislauf gepumpt werden, wobei eine Flussrichtung in Figur 1 symbolisch mit der Bezugsziffer 120 bezeichnet ist. Der Kreislauf kann insbesondere durch eine Pumpe 122 aufrechterhalten werden, welche über eine Entnahmeleitung 124 keimarme Flüssigkeit aus dem Behälter 116 entnimmt, diese durch das Rohrleitungssystem 118 pumpt und anschließend über eine Zuleitung 126, welche beispielsweise in einer Sprühdüse mündet, dem Behälter 116 wieder zuführt.

Zur Entnahme der keimarmen Flüssigkeit aus dem Rohrleitungssystem 118 weist das Leitungssystem 110 insbesondere mindestens einen Abzweig 128 auf, welcher beispielsweise als T-Stück ausgestaltet sein kann. Dieser Abzweig 128 ist direkt oder indirekt mit der Entnahmevorrichtung 112 verbunden. Zwischen der Entnahmevorrichtung 112 und dem Abzweig 128 kann mindestens ein Ventil 130 angeordnet sein, beispielsweise ein Blockventil. Dieses mindestens eine Ventil kann beispielsweise ein erstes Ventil 132 zum Verschließen des Abzweigs 128 aufweisen. Weiterhin kann zwischen dem Abzweig 128 und der Entnahmevorrichtung 112 mindestens eine Zufuhr 134 vorgesehen sein und in eine Verbindung zwischen der Entnahmevorrichtung 112 und dem Abzweig 128 einmünden. Diese Zufuhr 134 kann durch ein zweites Ventil 136 verschließbar sein. Dieses zweite Ventil 136 kann mit dem ersten Ventil 132 zu dem Ventil 130, beispielsweise dem Blockventil, zusammengefasst sein, kann jedoch auch getrennt von dem ersten Ventil 132 ausgebildet sein.

Auf einer von dem Rohrleitungssystem 118 abgewandten Seite 138 der Wand 114, welche auch als Benutzerseite bezeichnet werden kann, ist die nachfolgend noch näher beschriebene Entnahmevorrichtung 112 vorgesehen, welche an einem Ende mit dem Abzweig 128 verbunden ist. An ihrem gegenüberliegenden Ende kann die Entnahmevorrichtung 112, beispielsweise wiederum über ein Ventil 140, mit einem Abfluss 142 verbunden sein. Zwischen dem Ventil 140 und dem Abfluss 142 kann weiterhin mindestens ein Kondensatableiter 144 vorgesehen sein.

In den Figuren 2 bis 4 sind mögliche Einzelheiten der Entnahmevorrichtung 112 in einer Schnittdarstellung gezeigt. Die Entnahmevorrichtung 112 ist beispielsweise, wie in Figur 4 gezeigt, vertikal montiert und ist insgesamt näherungsweise U-förmig ausgestaltet.

Die Entnahmevorrichtung 112 weist einen Entnahmestutzen 146 auf, welcher an seinem oberen Ende 148 mit dem Ventil 132 und dem Abzweig 128 verbunden ist und somit mit keimarmer Flüssigkeit beaufschlagt werden kann. An seinem unteren Ende 150 weist der Entnahmestutzen 146 einen Flansch 152 oder Kragen auf, an welchen sich ein Rohrende 154 anschließt. Dieses Rohrende 154 kann beispielsweise, wie in den Figuren 2 und 3 erkennbar, abgeschrägt ausgebildet sein, um ein Ablaufen von Tropfen zu erleichtern.

Die Entnahmevorrichtung 112 weist weiterhin einen Koppelbogen 156 auf. Dieser Koppelbogen 156 kann beispielsweise ebenfalls rohrförmig ausgestaltet sein und stellt einen Hohlraum 158 bereit, in welchen das Rohrende 154 hineinragt. Der Koppelbogen 156 kann beispielsweise, wie auch der Entnahmestutzen 146, L-förmig ausgestaltet sein und kann an seinem unteren Ende 160, beispielsweise wiederum über einen Flansch 162, mit dem Abfluss 142 koppelbar sein. An seinem oberen Ende 164 kann der Koppelbogen 156 beispielsweise einen Einsatz 166 mit einem Außengewinde 168 aufweisen. Dieses Außengewinde 168 kann beispielsweise über eine in Figur 3 erkennbare Überwurfmutter 170 mit dem Flansch 152 verbunden werden.

Das Rohrende 154 ragt in den Hohlraum 158, der auch als Innenraum bezeichnet wird, hinein. Der Innenraum 158 ist derart dimensioniert, dass sich zwischen einer Innenwand 172 des Koppelbogens 156 und einer Außenseite 174 des Rohrendes 154 ein Ringspalt 176 ausbildet. Dementsprechend ist das Rohrende 154 frei in dem Innenraum 158 angeordnet, so dass eine Stirnseite 178, eine Innenseite 180 und die Außenseite 174 des Rohrendes 154 für Reindampf frei zugänglich sind und von Reindampf umspült werden können.

Für eine Vorbereitung einer Entnahme kann dementsprechend beispielsweise zunächst das erste Ventil 132 geschlossen werden und das zweite Ventil 136 geöffnet werden, so dass Reindampf über die Zufuhr 134 in die Entnahmevorrichtung 112 gelangen kann. Im Innenraum 158 des Koppelbogens 156 umspült dieser Reindampf das Rohrende 154 und sterilisiert die Außenseite 174, die Stirnseite 178 und die Innenseite 180. Über das Ventil 140 und den Kondensatableiter 144 kann der Reindampf anschließend abgeleitet werden.

Nach erfolgter Sterilisation kann auch das zweite Ventil 136 geschlossen werden und so die Zufuhr von Reindampf unterbrochen werden. Durch Lösen der Überwurfmutter 170 kann anschließend der Koppelbogen 156 von dem Entnahmestutzen 146 entfernt werden, so dass der in Figur 3 gezeigte Zustand mit freiliegendem Rohrende 154 entsteht. Anschließend kann das Ventil 132 geöffnet werden, und keimarme Flüssigkeit kann aus dem Entnahmestutzen 146 entnommen werden.

Wie in Figur 2 weiterhin gezeigt, kann der Koppelbogen 156 insbesondere thermisch isoliert ausgestaltet sein. So kann der Entnahmestutzen insbesondere doppelwandig ausgebildet sein, wie in Figur 2 durch die Bezugsziffer 182 symbolisch dargestellt.

In Figur 4 ist die Entnahmevorrichtung 112 in einer perspektivischen Darstellung in einem an die Wand 114 montierten Zustand gezeigt. Hierbei ist erkennbar, dass der Flansch 162 des Koppelbogens 156 beispielsweise ebenfalls über eine Überwurfmutter 184 fixiert werden kann. Weiterhin ist erkennbar, dass unterhalb der Entnahmevorrichtung 112 beispielsweise ein Spülbecken 186 vorgesehen sein kann, um auslaufende keimarme Flüssigkeit aufzunehmen. Weiterhin ist erkennbar, dass die Entnahmevorrichtung 112 weiterhin mindestens einen elektrischen Kontakt 188 aufweisen kann, welcher beispielsweise starr ausgebildet sein kann und welcher beispielsweise mit einer in Figur 4 symbolisch angedeuteten Steuerung 190 verbindbar sein kann. Diese Steuerung kann beispielsweise mit dem Ventil 130 und optional auch mit dem Ventil 140 verbunden sein und kann eine Entnahme ansteuern. Insbesondere kann eine Dampfbeaufschlagung durch die Steuerung 190 steuerbar sein. Beispielsweise kann die Steuerung 190 erkennen, ob der elektrische Kontakt 188 angekoppelt ist, was nur erfolgt, wenn der Koppelbogen 156 sich in dem in Figur 4 dargestellten montierten Zustand befindet. Nur in diesem Zustand kann Reindampf in den Koppelbogen 156 eingeleitet werden. Auf diese Weise kann die Steuerung 190 beispielsweise verhindern, dass Bedienpersonal durch Reindampf verletzt wird, wenn der Koppelbogen 156 nicht auf den Entnahmestutzen 146 aufgesetzt ist.

Die Entnahmevorrichtung 112 kann insbesondere ganz oder teilweise aus Edelstahl hergestellt sein. Besonders bevorzugt ist es dabei, wenn eine besonders geringe Oberflächenrauigkeit vorliegt, so dass eine Keimanhaftung auf den Oberflächen des Edelstahls erschwert wird. Beispielsweise kann der Edelstahl eine Oberflächenrauigkeit RA ≤ 0,8 Mikrometern oder RA < 0,8 Mikrometern oder weniger aufweisen.

### Bezugszeichenliste

- 110: Leitungssystem
- 112: Entnahmevorrichtung
- 114: Wand
- 116: Behälter
- 118: Rohrleitungssystem
- 120: Flussrichtung
- 122: Pumpe
- 124: Entnahmeleitung
- 126: Zuleitung
- 128: Abzweig
- 130: Ventil
- 132: erstes Ventil
- 134: Zufuhr für Reindampf
- 136: zweites Ventil
- 138: Benutzerseite
- 140: Ventil
- 142: Abfluss
- 144: Kondensatableiter
- 146: Entnahmestutzen
- 148: oberes Ende
- 150: unteres Ende
- 152: Flansch
- 154: Rohrende
- 156: Koppelbogen
- 158: Hohlraum, Innenraum
- 160: unteres Ende
- 162: Flansch
- 164: oberes Ende
- 166: Einsatz
- 168: Außengewinde
- 170: Überwurfmutter
- 172: Innenwand
- 174: Außenseite
- 176: Ringspalt
- 178: Stirnseite
- 180: Innenseite
- 182: doppelwandige Ausgestaltung
- 184: Überwurfmutter
- 186: Spülbecken
- 188: elektrischer Kontakt
- 190: Steuerung

## Patentansprüche

1. Leitungssystem (110) zur Bereitstellung mindestens einer keimarmen Flüssigkeit, umfassend:
- mindestens ein Rohrleitungssystem (118) für die keimarme Flüssigkeit, wobei das Rohrleitungssystem (118) ringförmig ausgestaltet ist und mindestens eine Pumpe (122) aufweist,
- mindestens eine Entnahmevorrichtung (112) zur Entnahme mindestens einer keimarmen Flüssigkeit aus dem Leitungssystem (110), umfassend mindestens einen Entnahmestutzen (146), weiterhin umfassend mindestens einen Koppelbogen (156), wobei der Koppelbogen (156) mit dem Entnahmestutzen (146) lösbar verbunden ist, wobei mindestens ein Rohrende (154) des Entnahmestutzens (146) in einen Innenraum (158) des Koppelbogens (156) hineinragt und in dem Koppelbogen (156) auf seiner Innenseite (180), seiner Außenseite (174) und seiner Stirnseite (178) mit Reindampf sterilisierbar ist, wobei das Rohrleitungssystem (118) mindestens einen Abzweig (128) aufweist, wobei der Abzweig (128) mit der Entnahmevorrichtung (112) verbunden ist, wobei zwischen dem Abzweig (128) und der Entnahmevorrichtung (112) mindestens eine Zufuhr (134) für Reindampf angeordnet ist, so dass Reindampf von der Zufuhr (134) durch den Entnahmestutzen (146) in den Koppelbogen (156) leitbar ist, um dort das Rohrende (154) des Entnahmestutzens (146) zu sterilisieren,
- mindestens einen Behälter (116) zur Aufnahme der keimarmen Flüssigkeit, wobei der Behälter (116) mit dem Rohrleitungssystem (118) verbunden ist, wobei die Pumpe (122) des Rohrleitungssystems (118) eingerichtet ist, um die keimarme Flüssigkeit in einem Kreislauf durch das Rohrleitungssystem (118) und den Behälter (116) zu pumpen.

2. Leitungssystem (110) nach dem vorhergehenden Anspruch, wobei das Rohrende (154) des Entnahmestutzens (146) ringförmig von einer Innenwand (172) des Koppelbogens (156) umschlossen ist, wobei das Rohrende (154) von der Innenwand (172) beabstandet ist, so dass sich ein Ringspalt (176) zwischen der Innenwand (172) und dem Rohrende (154) ausbildet, in welchen der Reindampf eindringen kann.

3. Leitungssystem (110) nach einem der vorhergehenden Ansprüche, wobei der Entnahmestutzen (146) rohrförmig ausgestaltet ist und einen Innendurchmesser aufweist, wobei der Koppelbogen (156) rohrförmig ausgestaltet ist und einen Innendurchmesser aufweist.

4. Leitungssystem (110) nach einem der vorhergehenden Ansprüche, wobei der Koppelbogen (156) zumindest teilweise doppelwandig ausgestaltet ist.

5. Leitungssystem (110) nach einem der vorhergehenden Ansprüche, wobei der Entnahmestutzen (146) L-förmig ausgestaltet ist und wobei der Koppelbogen (156) L-förmig ausgestaltet ist, so dass die Entnahmevorrichtung (112) insgesamt, mit dem Entnahmestutzen (146) und dem Koppelbogen (156) in verbundenem Zustand, U-förmig ausgestaltet ist.

6. Leitungssystem (110) nach einem der vorhergehenden Ansprüche, wobei die Stirnseite (178) des Rohrendes (154) des Entnahmestutzens (146) schräg gegenüber einer Rohrachse des Rohrendes (154) verläuft.

7. Leitungssystem (110) nach einem der vorhergehenden Ansprüche, wobei der Koppelbogen (156) als Rohrstück ausgestaltet ist, wobei eine erste Öffnung des Rohrstücks das Rohrende (154) des Entnahmestutzens (146) aufnimmt und wobei eine zweite Öffnung des Rohrstücks mit Reindampf beaufschlagbar ist, so dass Reindampf durch das Rohrstück von der zweiten Öffnung zu der ersten Öffnung gelangen kann und dort das Rohrende (154) des Entnahmestutzens (146) sterilisieren kann.

8. Leitungssystem (110) nach dem vorhergehenden Anspruch, wobei das Rohrstück L-förmig gebogen ist.

9. Leitungssystem (110) nach einem der vorhergehenden Ansprüche, wobei der Koppelbogen (156) mit dem Entnahmestutzen (146) über eine Schraubverbindung lösbar verbunden ist.

10. Leitungssystem (110) nach einem der vorhergehenden Ansprüche, wobei das Rohrende (154) des Entnahmestutzens (146) eine Oberflächenrauigkeit RA ≤ 0,8 Mikrometern aufweist.

11. Leitungssystem (110) nach einem der vorhergehenden Ansprüche, wobei die Entnahmevorrichtung (112) weiterhin einen mit dem Koppelbogen (156) verbundenen elektrischen Kontakt (188) zur Verbindung mit einer Steuerung (190) und zur Erkennung eines Zustandes, in welchem der Koppelbogen (156) mit dem Entnahmestutzen (146) verbunden ist, aufweist.

12. Leitungssystem (110) nach einem der vorhergehenden Ansprüche, wobei das Leitungssystem (110) weiterhin mindestens eine Steuerung (190) aufweist, wobei die Steuerung (190) über mindestens einen elektrischen Kontakt (188) mit dem Koppelbogen (156) verbindbar ist und eingerichtet ist, um zu erkennen, ob der Koppelbogen (156) mit dem Entnahmestutzen (146) verbunden ist, wobei die Steuerung (190) weiterhin eingerichtet ist, um nur dann Reindampf bereitzustellen, wenn der Koppelbogen (156) mit dem Entnahmestutzen (146) verbunden ist, und andernfalls eine Bereitstellung von Reindampf zu verhindern.

13. Leitungssystem (110) nach einem der vorhergehenden Ansprüche, wobei der Koppelbogen (156) an einem dem Entnahmestutzen (146) abgewandten Ende (160) mit mindestens einem Abfluss (142) verbunden ist.

14. Verfahren zur Entnahme mindestens einer keimarmen Flüssigkeit aus einem Leitungssystem (110), umfassend die folgenden Schritte:
a) Bereitstellen eines Leitungssystems (110) nach einem der vorhergehenden betreffenden Ansprüche;
b) Einleiten von Reindampf in den Koppelbogen (156) und Sterilisieren des Rohrendes (154) des Entnahmestutzens (146) in dem Innenraum (158) des Koppelbogens (156) durch Umspülen des Rohrendes (154) mit dem Reindampf;
c) Lösen und Entfernen des Koppelbogens (156) von dem Entnahmestutzen (146); und
d) Entnahme der keimarmen Flüssigkeit aus dem Entnahmestutzen (146).

## Claims

1. A conduit system (110) for providing at least one low-germ liquid, comprising:
- at least one ductwork system (118) for the low-germ liquid, wherein the ductwork system (118) is of annular design and has at least one pump (122),
- at least one removal device (112) for removing at least one low-germ liquid from the conduit system (110), comprising at least one removal connection piece (146), further comprising at least one coupling bend (156), wherein the coupling bend (156) is releasably connected to the removal connection piece (146), wherein at least one pipe end (154) of the removal connection piece (146) projects into an interior (158) of the coupling bend (156) and can be sterilized with pure steam in the coupling bend (156) on its inner side (180), its outer side (174) and its end side (178), wherein the ductwork system (118) has at least one branch (128), wherein the branch (128) is connected to the removal device (112), wherein at least one supply (134) for pure steam is arranged between the branch (128) and the removal device (112), such that pure steam can be conducted from the supply (134) through the removal connection piece (146) into the coupling bend (156) in order to sterilize the pipe end (154) of the removal connection piece (146) there,
- at least one container (116) for receiving the low-germ liquid, wherein the container (116) is connected to the ductwork system (118), wherein the pump (122) of the ductwork system (118) is configured to pump the low-germ liquid in a circuit through the ductwork system (118) and the container (116).

2. The conduit system (110) according to the preceding claim, wherein the pipe end (154) of the removal connection piece (146) is surrounded annularly by an inner wall (172) of the coupling bend (156), wherein the pipe end (154) is spaced apart from the inner wall (172), such that an annular gap (176), into which the pure steam is able to penetrate, is formed between the inner wall (172) and the pipe end (154).

3. The conduit system (110) according to either one of the preceding claims, wherein the removal connection piece (146) is of tubular design and has an inner diameter, wherein the coupling bend (156) is of tubular design and has an inner diameter.

4. The conduit system (110) according to any one of the preceding claims, wherein the coupling bend (156) is of at least partially double-walled design.

5. The conduit system (110) according to any one of the preceding claims, wherein the removal connection piece (146) is of L-shaped design and wherein the coupling bend (156) is of L-shaped design, such that the removal device (112) as a whole, with the removal connection piece (146) and the coupling bend (156) in the connected state, is of U-shaped design.

6. The conduit system (110) according to any one of the preceding claims, wherein the end side (178) of the pipe end (154) of the removal connection piece (146) runs obliquely with respect to a pipe axis of the pipe end (154).

7. The conduit system (110) according to any one of the preceding claims, wherein the coupling bend (156) is designed as a pipe piece, wherein a first opening of the pipe piece receives the pipe end (154) of the removal connection piece (146) and wherein pure steam can be applied to a second opening of the pipe piece, such that pure steam can pass through the pipe piece from the second opening to the first opening and can sterilize the pipe end (154) of the removal connection piece (146) there.

8. The conduit system (110) according to the preceding claim, wherein the pipe piece is of L-shaped design.

9. The conduit system (110) according to any one of the preceding claims, wherein the coupling bend (156) is releasably connected to the removal connection piece (146) via a screw connection.

10. The conduit system (110) according to any one of the preceding claims, wherein the pipe end (154) of the removal connection piece (146) has a surface roughness RA ≤ 0.8 micrometers.

11. The conduit system (110) according to any one of the preceding claims, wherein the removal device (112) further has an electrical contact (188), which is connected to the coupling bend (156), for connection to a controller (190) and for detecting a state in which the coupling bend (156) is connected to the removal connection piece (146).

12. The conduit system (110) according to any one of the preceding claims, wherein the conduit system (110) further has at least one controller (190), wherein the controller (190) can be connected to the coupling bend (156) via at least one electrical contact (188) and is configured to detect whether the coupling bend (156) is connected to the removal connection piece (146), wherein the controller (190) is further configured to provide pure steam only when the coupling bend (156) is connected to the removal connection piece (146), and otherwise to prevent the provision of pure steam.

13. The conduit system (110) according to any one of the preceding claims, wherein the coupling bend (156) is connected to at least one outflow (142) at an end (160) facing away from the removal connection piece (146).

14. A method for removing at least one low-germ liquid from a conduit system (110), comprising the following steps:
a) providing a conduit system (110) according to any one of the respective preceding claims;
b) introducing pure steam into the coupling bend (156) and sterilizing the pipe end (154) of the removal connection piece (146) in the interior (158) of the coupling bend (156) by flushing the pipe end (154) with the pure steam;
c) releasing and removing the coupling bend (156) from the removal connection piece (146); and
d) removing the low-germ liquid from the removal connection piece (146).

## Revendications

1. Système de conduite (110) pour la fourniture d'au moins un liquide pauvre en germes, comprenant :
- au moins un système de conduite tubulaire (118) pour le liquide pauvre en germes, dans lequel le système de conduite tubulaire (118) est de configuration annulaire et présente au moins une pompe (122),
- au moins un dispositif de prélèvement (112) pour le prélèvement d'au moins un liquide pauvre en germes du système de conduite (110), comprenant au moins une tubulure de prélèvement (146), comprenant en outre au moins un coude de couplage (156), dans lequel le coude de couplage (156) est relié amovible à la tubulure de prélèvement (146), dans lequel au moins une extrémité de tube (154) de la tubulure de prélèvement (146) fait saillie dans un espace interne (158) du coude de couplage (156) et peut être stérilisée dans le coude de couplage (156) sur son côté interne (180), son côté externe (174) et son côté frontal (178) avec de la vapeur pure, dans lequel le système de conduite tubulaire (118) présente au moins une bifurcation (128), dans lequel la bifurcation (128) est reliée au dispositif de prélèvement (112), dans lequel entre la bifurcation (128) et le dispositif de prélèvement (112), au moins une alimentation (134) pour la vapeur pure est agencée, de sorte que la vapeur pure peut être conduite de l'alimentation (134) à travers la tubulure de prélèvement (146) dans le coude de couplage (156), afin d'y stériliser l'extrémité de tube (154) de la tubulure de prélèvement (146),
- au moins un récipient (116) pour la réception du liquide pauvre en germes, dans lequel le récipient (116) est relié au système de conduite tubulaire (118), dans lequel la pompe (122) du système de conduite tubulaire (118) est destinée à pomper le liquide pauvre en germes dans un circuit à travers le système de conduite tubulaire (118) et le récipient (116).

2. Système de conduite (110) selon la revendication précédente, dans lequel l'extrémité de tube (154) de la tubulure de prélèvement (146) est entourée de manière annulaire par une paroi interne (172) du coude de couplage (156), dans lequel l'extrémité de tube (154) est espacée de la paroi interne (172) de sorte qu'une fente annulaire (176) est réalisée entre la paroi interne (172) et l'extrémité de tube (154), dans laquelle la vapeur pure peut pénétrer.

3. Système de conduite (110) selon l'une des revendications précédentes, dans lequel la tubulure de prélèvement (146) a une configuration tubulaire et présente un diamètre intérieur, dans lequel le coude de couplage (156) a une configuration tubulaire et présente un diamètre intérieur.

4. Système de conduite (110) selon l'une des revendications précédentes, dans lequel le coude de couplage (156) a une configuration au moins partiellement à double paroi.

5. Système de conduite (110) selon l'une des revendications précédentes, dans lequel la tubulure de prélèvement (146) a une configuration en forme de L et dans lequel le coude de couplage (156) a une configuration en forme de L, de sorte que le dispositif de prélèvement (112) dans son ensemble, avec la tubulure de prélèvement (146) et le coude de couplage (156) dans un état relié, a une configuration en forme de U.

6. Système de conduite (110) selon l'une des revendications précédentes, dans lequel la face frontale (178) de l'extrémité de tube (154) de la tubulure de prélèvement (146) se prolonge obliquement par rapport à un axe de tube de l'extrémité de tube (154).

7. Système de conduite (110) selon l'une des revendications précédentes, dans lequel le coude de couplage (156) a une configuration de pièce tubulaire, dans lequel une première ouverture de la pièce tubulaire reçoit l'extrémité de tube (154) de la tubulure de prélèvement (146) et dans lequel une seconde ouverture de la pièce tubulaire est alimentée en vapeur pure, de sorte que la vapeur pure peut arriver à travers la pièce tubulaire de la seconde ouverture à la première ouverture et peut y stériliser l'extrémité de tube (154) de la tubulure de prélèvement (146).

8. Système de conduite (110) selon la revendication précédente, dans lequel la pièce tubulaire est coudée en forme de L.

9. Système de conduite (110) selon l'une des revendications précédentes, dans lequel le coude de couplage (156) est relié amovible à la tubulure de prélèvement (146) par l'intermédiaire d'une liaison par vis.

10. Système de conduite (110) selon l'une des revendications précédentes, dans lequel l'extrémité de tube (154) de la tubulure de prélèvement (146) présente une rugosité de surface RA ≤ 0,8 micromètres.

11. Système de conduite (110) selon l'une des revendications précédentes, dans lequel le dispositif de prélèvement (112) présente en outre un contact électrique (188) relié au coude de couplage (156) pour une liaison avec une commande (190) et pour une reconnaissance d'un état dans lequel le coude de couplage (156) est relié à la tubulure de prélèvement (146).

12. Système de conduite (110) selon l'une des revendications précédentes, dans lequel le système de conduite (110) présente en outre au moins une commande (190), dans lequel la commande (190) peut être reliée par l'intermédiaire d'au moins un contact électrique (188) avec le coude de couplage (156) et est destinée à reconnaître si le coude de couplage (156) est relié à la tubulure de prélèvement (146), dans lequel la commande (190) est en outre destinée à fournir de la vapeur pure uniquement lorsque le coude de couplage (156) est relié à la tubulure de prélèvement (146) et dans le cas contraire pour empêcher une fourniture de vapeur pure.

13. Système de conduite (110) selon l'une des revendications précédentes, dans lequel le coude de couplage (156) est relié au niveau d'une extrémité (160) faisant face à la tubulure de prélèvement (146) avec au moins un déversement (142).

14. Procédé de prélèvement d'au moins un liquide pauvre en germes à partir d'un système de conduite (110), comprenant les étapes suivantes :
a) fourniture d'un système de conduite (110) selon l'une des revendications précédentes concernées ;
b) insertion de vapeur pure dans le coude de couplage (156) et stérilisation de l'extrémité de tube (154) de la tubulure de prélèvement (146) dans l'espace interne (158) du coude de couplage (156) par le biais de l'enveloppement de l'extrémité de tube (154) avec la vapeur pure ;
c) séparation et retrait du coude de couplage (156) de la tubulure de prélèvement (146) ; et
d) prélèvement du liquide pauvre en germes de la tubulure de prélèvement (146).
